# EUROPEAN PATENT SPECIFICATION

(11) **EP 4 748 369 B1**
(45) Date of publication and mention of the grant of the patent: **25.11.2026**
(21) Application number: 24383264.9
(22) Date of filing: 21.11.2024
(51) Int. Cl.: A61K 31/4375, A61K 33/243, A61K 9/08, A61K 47/02, A61K 45/06, A61P 35/00, A61K 9/00

(54) **PROTOBERBERINE DERIVATIVES FOR USE IN THE TREATMENT OF CANCER**
PROTOBERBERINDERIVATE ZUR VERWENDUNG BEI DER BEHANDLUNG VON KREBS
DÉRIVÉS DE PROTOBERBÉRINE POUR UNE UTILISATION DANS LE TRAITEMENT DU CANCER

(43) Date of publication of application: 27.05.2026
(73) Proprietor: Applied Research Using OMIC Sciences S.L., 08036 Barcelona (ES)
(72) Inventor: PLASENCIA CASTILLO, CARMEN, 08036 Barcelona (ES); GARCIA MUÑOZ, VIRGINIA, 08036 Barcelona (ES)
(74) Representative: Curell Suñol S.L.P.

(56) References cited:
- US-B2- 8 188 109

## Description

### Field of the invention

The invention relates to medical uses of a compound of general formula (I) 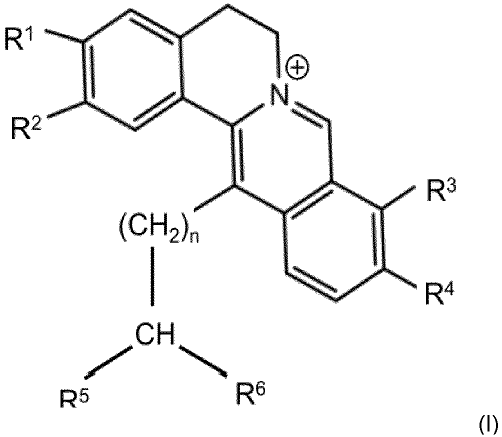 and of pharmaceutical compositions comprising said compound of general formula (I).

### State of the art

The primary indicators of success in advanced cancer treatment are overall survival and quality of life. Over the last three decades, improvements in these outcomes have been observed, with acceleration in treatment advancements over the years. However, despite significant progress in cancer therapy, numerous patients with advanced refractory, or resistant cancers still cannot be cured using currently available treatments. According to recent Global Cancer Observatory data, in 2022, cancer remains a leading cause of death, with nearly 2 million deaths from lung cancer, 1 million from colorectal cancer, and hundreds of thousands from other cancers like breast, stomach, and prostate cancers. In addition to these frequently occurring tumours, several less common tumours, such as mesothelioma which accounts for over 25,000 deaths, and often with fewer therapeutic options, require further consideration. This highlights the ongoing need for the development of new, more effective therapies, especially for patients with advanced, refractory, or resistant cancers.

Recent advancements in molecular biology have revealed complex regulatory networks that govern cancer progression. A key finding is the importance of complex secondary nucleic acid structures found in both DNA and RNA, which regulate the expression of genes involved in tumour progression and drug resistance. This opens the door to new therapeutic approaches targeting these structures.

One of the crucial pathways for cancer cell proliferation and survival is nucleotide metabolism. Compared to normal, non-proliferating cells, cancer cells are highly dependent on the *de novo* synthesis of nucleotides to produce sufficient DNA and RNA precursors necessary for growth. Thymidylate Synthase (TS) is a key rate-limiting enzyme involved in the *de novo* synthesis of thymidine monophosphate, an essential nucleotide required for DNA replication and repair. Recent studies also suggest that TS may serve as a regulator of cancer stemness, indicating its involvement in cancer cell de-differentiation in cancer diseases. The absence or inhibition of thymidylate synthesis not only hinders cell proliferation but also induces cell death, underscoring the therapeutic importance of this target. Overexpression of TS correlates with aggressive solid tumours, which are characterized by a highly invasive phenotype, chemoresistance, and poor prognosis in patients. This is the case, for example, in various solid tumour types, including non-small-cell lung cancer (NSCLC), breast cancer, ovarian cancer, prostate cancer, pancreatic cancer, colorectal cancer, gastric cancer, oesophageal squamous cell carcinoma (ESCC), and hepatocellular carcinoma (HCC), among others.

Considering this, chemotherapeutic agents targeting thymidylate biosynthesis and directly inhibiting the TS enzyme are currently employed in clinical settings for treating solid and haematological tumours, either as standalone treatments or in combination regimens. Classical TS inhibitors fall into two major categories: antimetabolites (such as 5-fluorouracil and capecitabine) and antifolates (such as methotrexate and pemetrexed). These agents compete with the natural substrate or the cofactor for binding to the dimeric form of the enzyme, thereby blocking its activity and inducing a state of thymidylate deficiency, which prevents cancer cells from proliferating. When used alone, these agents can effectively target rapidly dividing cells. However, their efficacy often increases when combined with other chemotherapeutic agents or targeted therapies. Combination therapies can help overcome resistance, enhance cancer cell death, and minimize the likelihood of relapse, offering a more comprehensive cancer treatment strategy.

Despite the utility of antimetabolites and antifolates, their use is not without limitations.

Primary challenges include toxicity to normal, rapidly dividing cells, leading to side effects such as myelosuppression, mucositis, and gastrointestinal toxicity. Moreover, cancer cells can develop resistance to these drugs over time through various mechanisms, including increased drug efflux, modifications to drug targets, or enhanced DNA repair capabilities. This resistance limits their long-term efficacy and necessitates the development of new strategies to improve effectiveness. Furthermore, there exists a narrow therapeutic window between efficacy and toxicity, necessitating careful dosing and monitoring to avoid severe adverse effects.

Therefore, there is the need to provide alternative treatments for cancer, preferably treatments for advanced and/or refractory and/or resistant tumours, that allow to overcome the above-mentioned problems. US 8 188 109 B2 discloses the use of a protoberberine derivative for the treatment of cancer, which is tested *in vitro* on various cancer cell lines, such as human ovarian cancer cell lines, and Human tumor cells (HeLa) from uterin cervix and HCTI 16 from colon carcinoma.

### Summary of the invention

It is a purpose of the invention to provide an effective treatment of cancer in a human subject according to claim 1.

This purpose is achieved by a compound of general formula (I) according to claim 1 for use according to claim 1.

It is another object of the invention to provide a pharmaceutical composition for use according to claim 1, wherein said pharmaceutical composition comprises a compound of general formula (I) according to claim 1, and wherein said pharmaceutical composition is administered to said human subject such that a therapeutically effective dose of said compound of general formula (I) is administered to said human subject.

The invention further includes a number of preferred features that are object of the dependent claims and the utility of which will be highlighted hereinafter in the detailed description of an embodiment of the invention.

In the context of the invention, the term "treatment" is used to refer to the administration of a compound of formula I, or of a medicament or pharmaceutical composition comprising it, to control the progression of the disease before or after the clinical signs have appeared. Control of the progression of the disease is understood as the beneficial or desired clinical results which include but are not limited to reduction of the symptoms, reduction of the duration of the disease, stabilization of pathological conditions (specifically avoiding additional impairment), delaying the progression of the disease, improving the pathological condition and remission (both partial and complete). The control of the progression of the disease also involves a prolongation of survival in comparison to the expected survival if the treatment was not applied.

In the context of the invention, the expression "a therapeutically effective dose" means a dose sufficient to cause an effective treatment of a pathology or condition, preferably, an effective treatment of a cancer. In the context of the invention, "an effective treatment of a cancer" means is a treatment that results in a clinically relevant sign of improvement of the pathology or condition such as the ones described above. In the case of a cancer disease, clinically relevant signs of improvement can be for example, but are not limited to, (i) reduction in tumour size, (ii) inhibition of tumour progression and metastatic spread, (iii) prolongation of patient survival, or (iv) improvement in overall response rate as determined by, preferably, the Response Evaluation Criteria in Solid Tumours (RECIST) guidelines. For example, the RECIST guidelines (version 1.1) available at DOI:10.1016/j.ejca.2008.10.026.

Preferably, said compound is administered intravenously, more preferably said compound is administered intravenously at least once a week.

Intravenous administration presents challenges both from a formulation point of view of the compound, as well as from an *in vivo* efficacy and a safety perspective, e.g. due to the risk of reduced efficacy due problems during preparation of the injectable formulation and/or to the risk and severity of side effects derived from the compound being delivered directly into the blood stream. To the best knowledge of the inventors, efficacious intravenous administration to treat cancer, preferably to treat solid tumours, has not yet been proved for a compound according to the invention. As shown in the examples below the compound according to the invention can safely achieve its therapeutical effect against a variety of cancers via this administration route, thus allow treatments that have the advantages of such administration route such as the compound rapidly reaching its target and the therapeutic effect often being achieved quickly.

In the context of the invention, the expression "administered intravenously", means that an "intravenous administration" ("i.v. administration") route, that is an administration via a vein of said human subject, is used. Preferably, said intravenous administration is by injection of said compound of general formula (I) according to the invention, or by injection of a medicament or a pharmaceutical composition comprising said compound of general formula (I) according to the invention.

Preferably, said therapeutically effective dose is a dose sufficiently to cause an effective treatment of cancer. More preferably, said therapeutically effective dose is a dose comprised between a Minimal Starting Dose (MSD) and the Maximum Tolerated Dose (MTD).

In the context of the invention, doses of the compound, including the Minimal Starting Dose (MSD) and the Maximal Tolerated Dose (MTD), can be expressed in "mg/kg of body weight of the subject to be treated" or "mg/m² body surface area of the subject to be treated". The correspondence between doses expressed in "mg/kg of body weight of the subject to be treated" and doses expressed in "mg/m² body surface area of the subject to be treated" is known by the skilled person in the art based on, for example, the relevant guidelines, for example *"*USFDA. Guidance for Industry: Estimating the Maximum Safe Starting Dose in Adult Healthy Volunteer. Rockville, MD: US Food and Drug Administration; 2005*".*

In the context of the invention, the minimal starting dose (MSD) can be the recommended starting dose. The recommended starting dose can be determined according to the ICH S9 guideline. For example, the recommended starting dose can be derived from one-tenth of the most severely toxic dose in 10% of the animals (STD10) in rodents, provided this does not cause severe irreversible toxicity in a non-rodent species, or is derived from one-sixth of the highest non-severely toxic dose (HNSTD) in non-rodents, adjusted for interspecies scaling. Adjustment for interspecies scaling can be done using the relevant guidelines, for example *"*USFDA. Guidance for Industry: Estimating the Maximum Safe Starting Dose in Adult Healthy Volunteer. Rockville, MD: US Food and Drug Administration; 2005*".* For example, the Minimal Starting Dose (MSD) can be 9 mg/m² of body surface area of a human subject as determined based on the one-tenth of the STD10 dose in rats.

Preferably, minimal starting dose (MSD) is the recommended starting dose according to the relevant guidelines, for example according to the *"*ICH guideline S9 on non-clinical evaluation for anticancer pharmaceuticals, first published on the 11/02/2013 and last updated on the 11/02/2013*".* More preferably, the Minimal Starting Dose (MSD) is derived from one-tenth of the most severely toxic dose in 10% of the animals (STD10) in rodents, provided this does not cause severe irreversible toxicity in a non-rodent species, or alternatively, if one-tenth of the STD10 causes severe toxicity in non-rodents, the Minimal Starting Dose (MSD) is derived from one-sixth of the non-rodent highest non-severely toxic dose (HNSTD), adjusted for interspecies scaling. In a further preferred embodiment, the Minimal Starting Dose (MSD) is 9 mg/m2 of body surface area of a human subject.

In the context of the invention, the Maximal Tolerated Dose is the highest dose at which no more than one in six patients experience a Dose Limiting Toxicity (DLT) related to the compound. For example, the Maximal Tolerated Dose (MTD) can be determined in a Phase 1 clinical trial starting from said minimal starting dose (MSD) by carrying out a dose escalation trial, for example, with two or more expansion cohorts to investigate the safety, tolerability, pharmacokinetics, pharmacodynamics, and antitumor activity of the compound in patients with cancer.

In the context of the invention, a "dose escalation trial" means, for example, a trial wherein patients are enrolled sequentially into escalating dose cohorts and will continue receiving the compound at the tested dose until disease progression, unacceptable toxicity, withdrawal of consent or otherwise as specified in the investigational medicinal product (IMP) discontinuation criteria associated with said trial. For example, patients in said dose cohort can receive said compound or a composition comprising said compound, preferably by intravenous (IV) administrations, at the same dose in a dosing interval of at least once a week, preferable twice a week, for several weeks in cycles, preferably for four weeks per cycle consecutively without interruption, except when necessary to manage adverse events (AEs). Dose escalation may continue beyond, until the Maximal Tolerated Dose (MTD) can be defined, for example, based on safety, preliminary efficacy, PK and PD data, based on the recommendations of the Safety Review Committee (SRC) overseeing the trial. For example, in determining the Maximal Tolerated Dose (MTD), a Dose Limiting Toxicity (DLT) can be determined during the escalation trial based on the National Cancer institute - Common Terminology Criteria for Adverse Events (NCI-CTCAE) version 5.0 published on the 27 November 2017.

Preferably, the Maximal Tolerated Dose is defined as the highest dose at which no more than one in six patients experience a Dose Limiting Toxicity related to the compound. More preferably, a Dose Limiting Toxicity (DLT) is determined during the escalation trial based on the National Cancer institute - Common Terminology Criteria for Adverse Events (NCI-CTCAE) version 5.0 published on the 27 November 2017. Even more preferably, the Maximum Tolerated Dose (MTL) is determined in a Phase 1 clinical trial starting from said Minimal Starting Dose (MSD) by carrying out a dose escalation trial and is defined based on safety, preliminary efficacy, PK and PD data, based on the recommendations of the Safety Review Committee (SRC) overseeing the trial.

Preferably, said cancer comprises or consists of a solid tumour.

In the context of the invention, the expression "a solid tumour" means a tumour that forms a mass, typically an aberrant or abnormal mass, in a tissue. Solid tumours initially develop in a tissue at a specific location and can potentially lead to tumoral cells circulating in the blood stream that, if they establish themselves in another location, can lead to the formation of other tumours. The original tumour is known as primary tumour whereas the newly formed tumours from said tumoral cells circulating, are known as secondary tumours.

Preferably, said cancer comprising or consisting of a solid tumour is mesothelioma, preferably pleural mesothelioma or peritoneal mesothelioma, and more preferably pleural mesothelioma.

Preferably, said cancer comprising or consisting of a solid tumour is a lung cancer, preferably wherein said lung cancer is small cell lung cancer or non-small-cell lung cancer, more preferably wherein said lung cancer is a non-small cell lung cancer subtype, and even more preferably wherein said lung cancer is a non-small cell lung cancer subtype selected from the group consisting of adenocarcinoma or a squamous cell carcinoma.

In the context of the invention, the term "non-small-cell lung cancer" or abbreviated "NSCLC" refers to a group of heterogenous cancer diseases that share their prognosis and management. Although tumour classification has evolved rapidly in the last years, the term "non-small-cell lung cancer" includes cancer comprising or consisting of at least a tumour selected from the group consisting of adenocarcinomas, squamous cell carcinomas (SCC), and large cell carcinomas (LCC).

The skilled person in the art knows how to classify tumours, particularly lung tumours following established criteria such as the World Health Organization (WHO) Classification of Tumours, 5th Edition, 2021. For example, classification particularly for lung tumours can be found in the WHO Classification of Tumours, 5th Edition, 2021 (ISBN-13 978-92-832-4506-3), Volume 5 related to Thoracic Tumours.

Preferably, said cancer comprising or consisting of a solid tumour is colorectal cancer.

Preferably, said cancer comprising or consisting of a solid tumour is ovarian cancer.

According to the invention, said cancer comprises or consists of an advanced tumour.

In the context of the invention, an "advanced tumour" is a tumour that is at a stage where it has either grown significantly in size, and/or it has spread to nearby tissues, and/or it has metastasized to distant parts of the body. Typically, advanced tumours are not susceptible to be treated by surgery, due to their size, level of spread, or level of metastasis found throughout the body of the patient.

According to the invention, an advanced tumour is a tumour that is at a stage where it has grown significantly in size, more preferably where it has grown to a size which makes it not susceptible to be treated by surgery, and/or a tumour that has spread to nearby tissues, and/or a tumour that has metastasized to distant parts of the body.

Preferably, said cancer comprises or consists of a poorly responsive tumour.

In the context of the invention, the expression "a poorly responsive tumour" refers to a tumour that shows a poor response to treatment, preferably to available treatments such as Standard of Care treatments for said tumour. In the context of the invention, "a poor response to treatment" is a response to treatment unable to control the progression of the cancer or tumour.

Poorly responsive tumours can be classified as refractory tumours, resistant tumours, and relapsed/recurrent tumours. Tumours can be at the same time refractory or resistant, and relapsed recurrent tumours.

Preferably, said poorly responsive tumour is selected from the group consisting of a refractory tumour, a resistant tumour, a relapsed/recurrent tumour, or a combination thereof.

Preferably, said cancer comprises or consists of a tumour selected from the group consisting of a refractory tumour, a resistant tumour, a relapsed/recurrent tumour, and a combination thereof.

In the context of the invention, "a refractory tumour" is a tumour nonresponding initially to therapy, preferably to the standard of care treatment. The standard of care treatment for a given tumour is the treatment that is preferred for said tumour as it has shown the most effective results in treating said tumour.

In the context of the invention, "a resistant tumour" is a tumour that acquires or has acquired a resistance over time to a particular treatment. Certain tumours can acquire resistance to a particular treatment, for example, since they start to develop specific mutations that confer them the ability to be resistant to said particular treatment. Other tumours can acquire resistance, for example, due to changes in the tumour microenvironment, which facilitates the survival of tumoral cells and limits the efficacy of said particular treatment.

In the context of the invention, "a relapsed/recurrent tumour" is a tumour that has relapsed, that means that has come back after having been considered in remission. Remission can include partial remission and complete remission.

Preferably, said cancer comprises or consists of an advanced refractory solid tumour or an advanced resistant solid tumour. More preferably, said cancer is selected from the group consisting of advanced refractory mesothelioma, advanced refractory lung cancer, advanced refractory colorectal cancer, advanced refractory ovarian cancer, advanced resistant mesothelioma, advanced resistant lung cancer, advanced resistant colorectal cancer, and advanced resistant ovarian cancer.

Preferably, said treatment is administered in combination with an additional cancer therapy. More preferably said additional cancer therapy is selected from the group consisting of chemotherapy, radiotherapy, immunotherapy, hormonal therapy, and a combination thereof. Even more preferably said additional cancer therapy is the cancer therapy considered the standard of care treatment for said cancer. In a further preferred embodiment, said treatment is administered in combination with cisplatin.

Preferably, said compound is a compound of formula (I) wherein R1 and R2 taken together are a methylenedioxy group, R3 is a methoxy group, R4 is a methoxy group, R5 is a phenyl group, and R6 is a phenyl group, and wherein n is 2.

Preferably, said compound is a compound of formula (I) wherein R1 and R2 taken together are a methylenedioxy group, R3 is a methoxy group, R4 is a hydroxy group, R5 is a phenyl group, and R6 is a phenyl group, and wherein n is 2.

Preferably, said compound is a compound of formula (I) wherein R1 and R2 taken together are a methylenedioxy group, R3 is a hydroxy group, R4 is a methoxy group, R5 is a phenyl group, and R6 is a phenyl group, and wherein n is 2.

Preferably, said compound is a compound of formula (I) wherein R1 is a methoxy group, R2 is a hydroxy group, R3 is a methoxy group, R4 is a methoxy group, R5 is a phenyl group, and R6 is a phenyl group, and wherein n is 2.

Preferably, said compound is a compound of formula (I) wherein R1 is a hydroxy group, R2 is a methoxy group, R3 is a methoxy group, R4 is a methoxy group, R5 is a phenyl group, and R6 is a phenyl group, and wherein n is 2.

In a preferred embodiment, the invention relates to a compound of general formula (I) 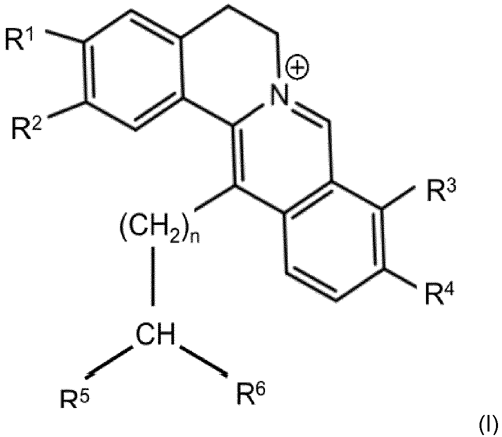 wherein R1 and R2 taken together are a methylenedioxy group, R3 is a methoxy group, R4 is a methoxy group, R5 is a phenyl group, and R6 is a phenyl group, and wherein n is 2, for use in the treatment of cancer in a human subject, wherein said compound of general formula (I) is administered intravenously in a therapeutically effective dose to said human subject, wherein said cancer comprises or consists of a solid tumour, and wherein said cancer comprises or consists of at least one selected from the group consisting of an advanced tumour, a refractory tumour, a resistant tumour, a relapsed/recurrent tumour, and any combination thereof. More preferably, wherein said cancer is an advanced solid tumour, or a refractory solid tumour, or a resistant solid tumour, or a relapsed/recurrent solid tumour, wherein said cancer is selected from the group consisting of pleural mesothelioma, peritoneal mesothelioma, small cell lung cancer, non-small-cell lung cancer, colorectal cancer, and ovarian cancer.

In a preferred embodiment, the invention relates to a compound of general formula (I) 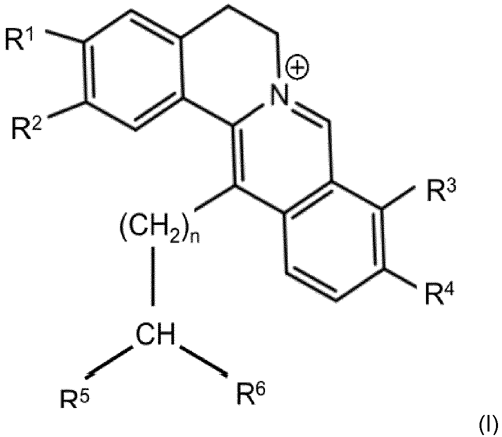 wherein R1 and R2 taken together are a methylenedioxy group, R3 is a methoxy group, R4 is a methoxy group, R5 is a phenyl group, and R6 is a phenyl group, and wherein n is 2, for use in the treatment of cancer in a human subject, wherein said compound of general formula (I) is administered intravenously in a therapeutically effective dose to said human subject, wherein said cancer comprises or consists of a solid tumour, and wherein said cancer comprises or consists of at least one selected from the group consisting of an advanced tumour, a refractory tumour, a resistant tumour, a relapsed/recurrent tumour, and any combination thereof, and wherein said treatment is administered in combination with an additional cancer therapy. More preferably wherein said additional cancer therapy is selected from the group consisting of chemotherapy, radiotherapy, immunotherapy, hormonal therapy, and a combination thereof. Even more preferably wherein said additional cancer therapy is the cancer therapy considered a standard of care treatment for said cancer, and wherein said cancer is selected from the group consisting of pleural mesothelioma, peritoneal mesothelioma, small cell lung cancer, non-small-cell lung cancer, colorectal cancer, and ovarian cancer.

Preferably, said pharmaceutical composition comprises a compound of general formula (I) according to claim 1.

Preferably, said pharmaceutical composition comprises a compound of formula (I) wherein R1 and R2 taken together are a methylenedioxy group, R3 is a methoxy group, R4 is a methoxy group, R5 is a phenyl group, and R6 is a phenyl group, and wherein n is 2.

Preferably, said pharmaceutical composition comprises a compound of formula (I) wherein R1 and R2 taken together are a methylenedioxy group, R3 is a methoxy group, R4 is a hydroxy group, R5 is a phenyl group, and R6 is a phenyl group, and wherein n is 2.

Preferably, said pharmaceutical composition comprises a compound of formula (I) wherein R1 and R2 taken together are a methylenedioxy group, R3 is a hydroxy group, R4 is a methoxy group, R5 is a phenyl group, and R6 is a phenyl group, and wherein n is 2.

Preferably, said pharmaceutical composition comprises a compound of formula (I) wherein R1 is a methoxy group, R2 is a hydroxy group, R3 is a methoxy group, R4 is a methoxy group, R5 is a phenyl group, and R6 is a phenyl group, and wherein n is 2.

Preferably, said pharmaceutical composition comprises a compound of formula (I) wherein R1 is a hydroxy group, R2 is a methoxy group, R3 is a methoxy group, R4 is a methoxy group, R5 is a phenyl group, and R6 is a phenyl group, and wherein n is 2.

Preferably, said pharmaceutical composition is a liquid pharmaceutical composition comprising a pharmaceutically acceptable vehicle such that said liquid pharmaceutical composition is isotonic and such that said liquid pharmaceutical composition has a physiologic pH value, preferably said pharmaceutically acceptable vehicle is a phosphate buffer.

Preferably, said pharmaceutical composition is in the form of a lyophilized powder for reconstitution and for injection in humans.

Preferably, said pharmaceutical composition is administered intravenously, more preferably said pharmaceutical composition is administered intravenously at least once a week.

Likewise, the invention also includes other features of detail illustrated in the detailed description of an embodiment of the invention and in the accompanying figures.

### Brief description of the drawings

Further advantages and features of the invention will become apparent from the following description, in which, without any limiting character, preferred embodiments of the invention are disclosed, with reference to the accompanying drawings in which:

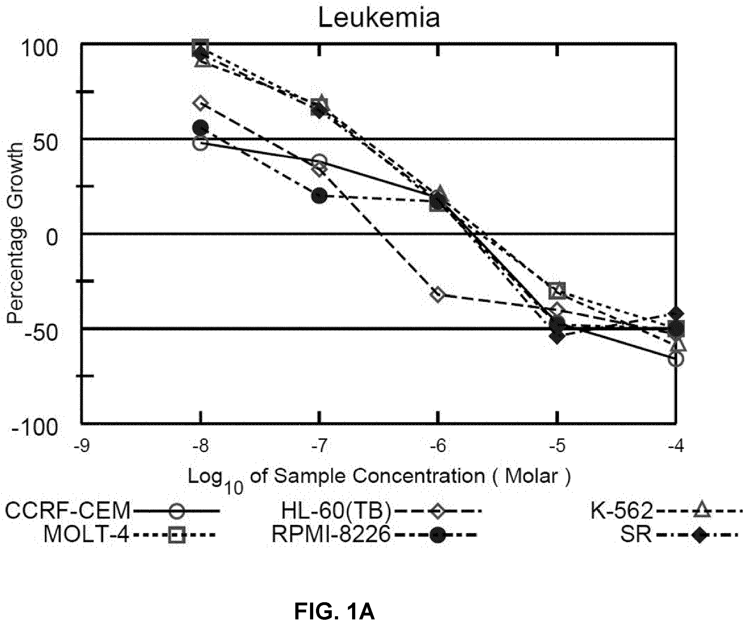

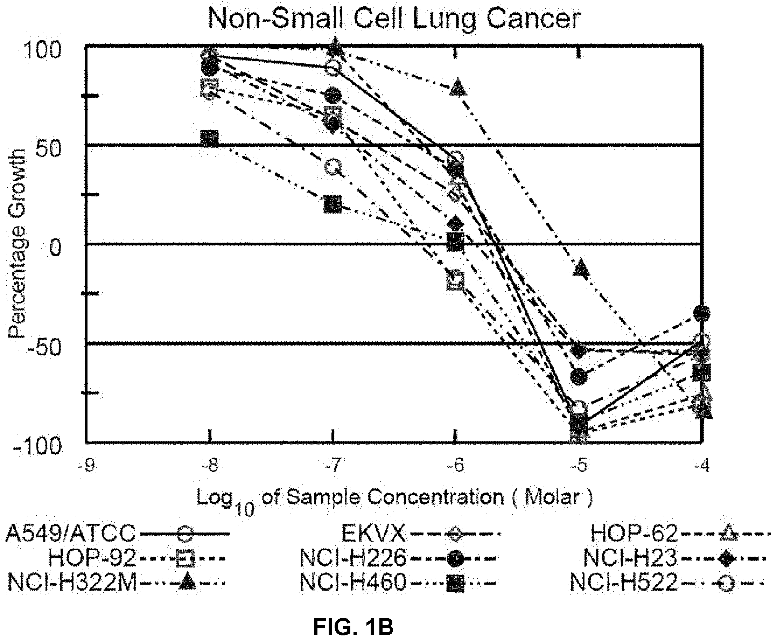

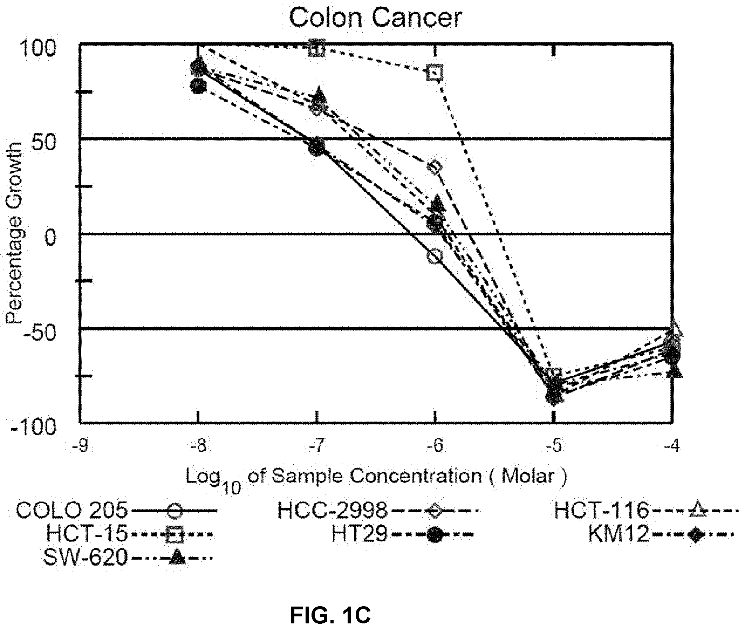

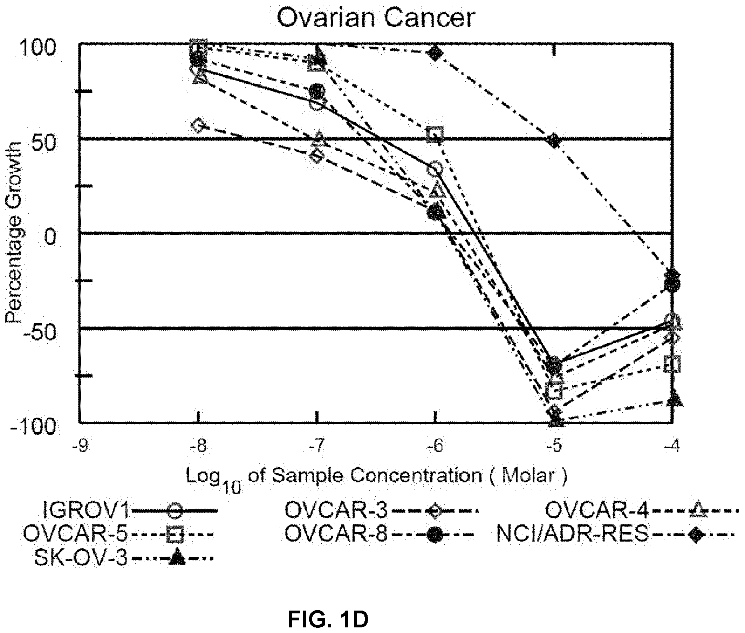

Figures 1A to 1D show a subset of the results and analysis obtained from a panel of cancer cell lines (NCI 60 Screening Methodology - NIH) treated with 13-(3,3-diphenylpropyl)-9,10-dimethoxy-5,6-dihydrobenzo[g]-1,3-benzodioxolo [5,6-a]quinolizinium chloride (Compound #1 salt).

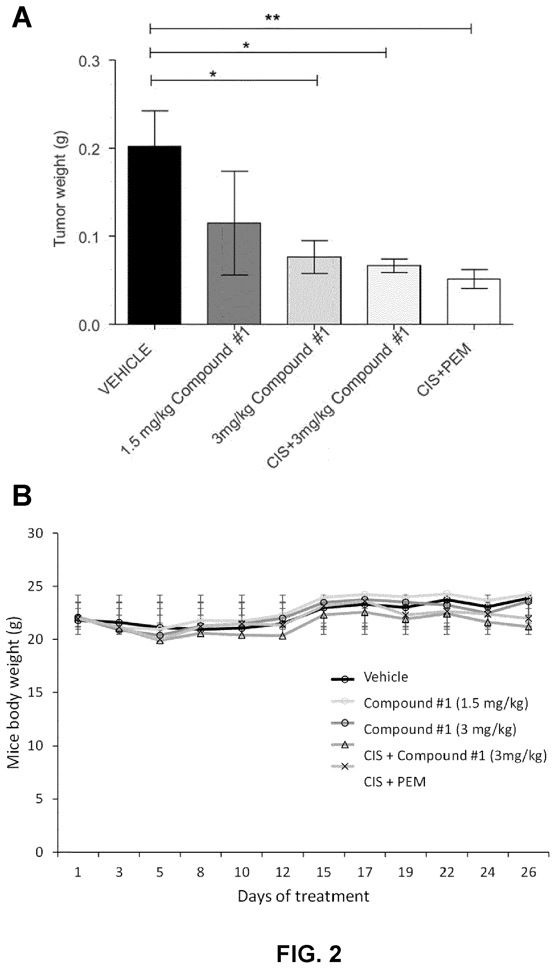

Figure 2 shows the antitumoral efficacy and safety of Compound #1 when administered intravenously alone or in combination with cisplatin on tumour growth (A) and animal body weight (B) in an experimental orthotopic model of mesothelioma. Values are represented as Mean ± SEM of 6 animals in each group. Statistical analysis was carried out by one way ANOVA followed by the Dunnett test. Asterisks indicate significant difference when treatment groups were compared with the Vehicle control group (group 1) *P=0.014; **P<0.005.

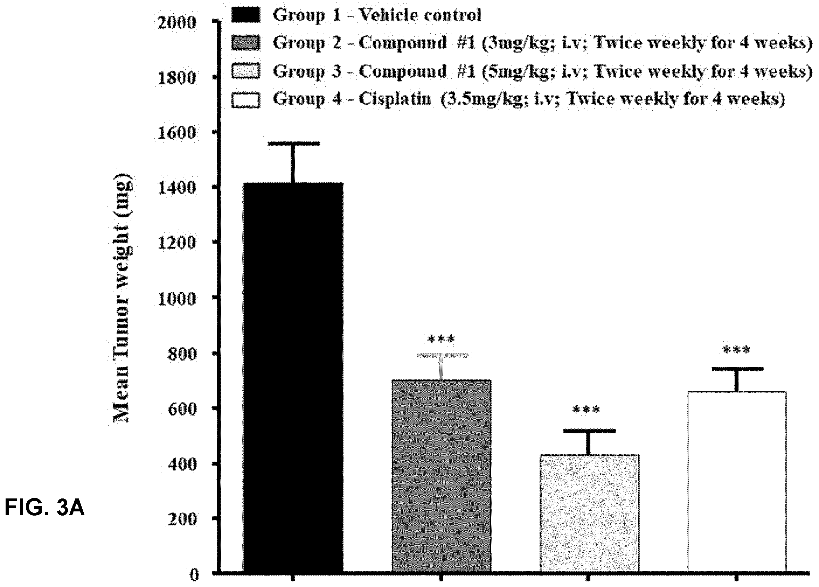

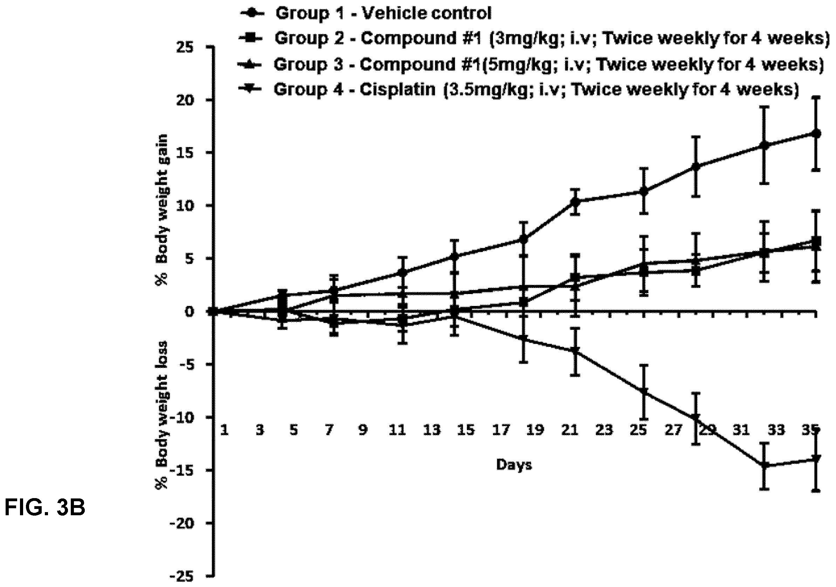

Figures 3A and 3B show the antitumoral efficacy (Fig. 3A) and safety (Fig. 3B) of Compound #1 when administered intravenously alone in a NSCLC Xenograft Model. Intravenous administration of cisplatin was performed for comparison. In Figure 3A values are represented as Mean ± SEM of 6 animals in each group. Statistical analysis was carried out by one way ANOVA followed by the Dunnett test. Asterisks indicate significant difference when treatment groups were compared with the Vehicle control group (group 1) ***P<0.001. In Figure 3B values are represented as Mean ± SEM of 6 animals in each group. Statistical analysis was carried out by Two-way ANOVA followed by Bonferroni test.

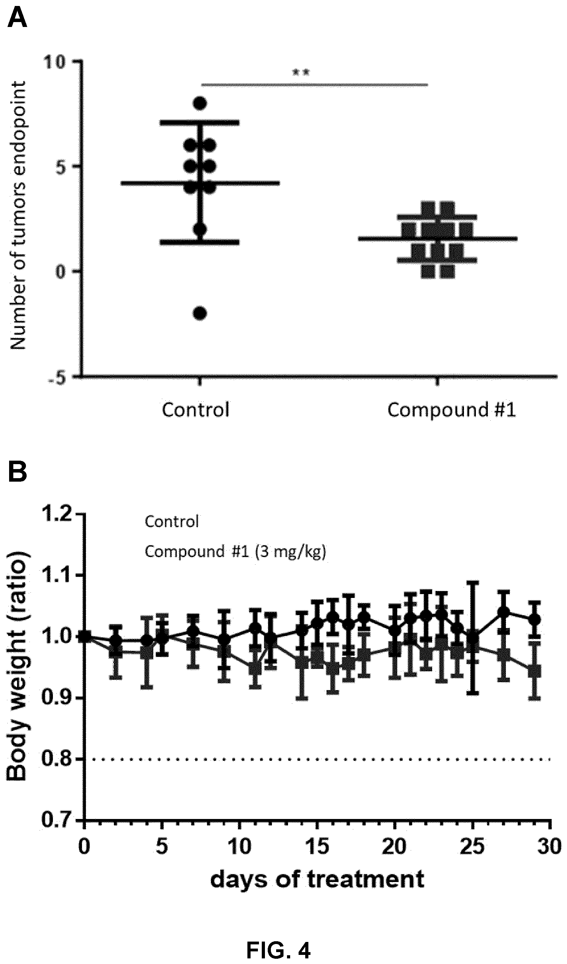

Figures 4A and 4B show the antitumoral efficacy (Fig. 4A) and safety (Fig. 4A) of Compound #1 in an AOM/DSS model for studying colorectal carcinogenesis based on the number of tumors counted after experiment termination (A) and body weight measurements throughout the course of the study period (B).

### Detailed description of embodiments of the invention

### Example 1

### Synthesis of berberine derivative 13-(3,3-diphenylpropyl)-9,10-dimethoxy-5,6-dihydrobenzo[g]-1,3-benzodioxolo [5,6-a]quinolizinium chloride (Compound #1 salt)

Compound 1# of formula shown below, for example in the form of its chloride salt, can be manufactured for the therapeutic uses according to the invention by the following method.

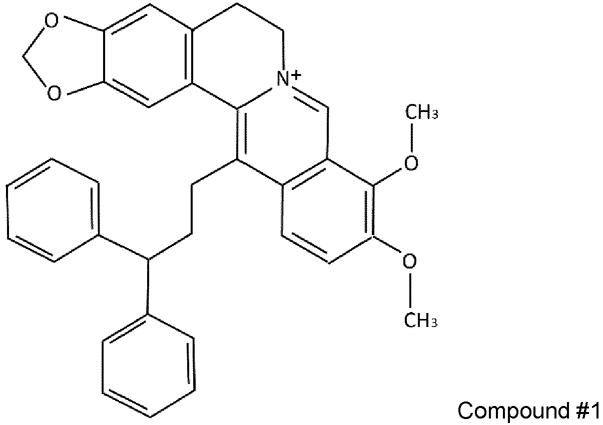

A solution of 3,3-diphenylpropanoic acid (10 g) in dry dichloromethane (70 mL) was cooled to 0°C, and 1,1'-carbonyldiimidazole (CDI) (8 g) was added portion-wise. The reaction mixture was stirred for 1 hour while maintaining the temperature at 0-10°C.

Diisobutylaluminum hydride (DIBAL-H, 1.0 M in dichloromethane, 93 mL) was added dropwise while keeping the reaction temperature below 10°C. After stirring for 2 hours, the reaction was quenched with an aqueous solution of tartaric acid. The organic phase was separated, dried, and concentrated to afford 3,3-diphenylpropanal, which was used in the next step without further purification. The reduction was successfully performed in 10 g scale and allowed obtaining and isolating 7 g of the aldehyde 3,3-diphenylpropanal.

Berberine hydrochloride (10 g) was suspended in isopropanol (200 mL) and treated with a sodium hydroxide solution (prepared from 5 g NaOH and 95 mL of purified water) at 20-30°C. Sodium borohydride (NaBH₄) (11 g) was added in portions while maintaining the temperature. After stirring for 1 hour, the mixture was cooled to 0-10°C and stirred for an additional 2 hours. The precipitated solid was filtered, washed with isopropanol, and dried under vacuum to obtain dihydroberberine. 7.82 g of dihydroberberine.

Dihydroberberine (3 g) and 3,3-diphenylpropanal (1.9 g) were dissolved in a mixture of isopropanol (24 mL) and acetic acid (6 mL). The reaction mixture was heated to reflux and stirred for 5 hours. After completion, the reaction mixture was allowed to cool to room temperature and stirred for an additional 12 hours. The solid product was filtered and washed with isopropanol, yielding the crude berberine derivative 13-(3,3-diphenylpro-pyl)-9,10-dimethoxy-5,6-dihydrobenzo[g]-1,3-benzodioxolo [5,6-a]quinolizinium chloride. 1.89 g of 13-(3,3-diphenylpropyl)-9,10-dimethoxy-5,6-dihydrobenzo[g]-1,3-benzodioxolo [5,6-a]quinolizinium chloride (Compound #1 salt) were obtained.

Compound 1#, for example in the form of its chloride salt, can be formulated in a liquid pharmaceutical composition comprising a pharmaceutically acceptable vehicle such that said liquid pharmaceutical composition is isotonic and such that said liquid pharmaceutical composition has a physiologic pH value, for example in a phosphate buffer.

Compound 1#, for example in the form of its chloride salt, can also be lyophilized to obtain a lyophilized powder suitable for reconstitution and for injection in human, preferably via intravenous route.

### Example 2

Figures 1A to 1D show a subset of the results and analysis of a panel of cancer cell lines (NCI 60 screening methodology) treated with the berberine derivative 13-(3,3-diphenylpropyl)- 9,10-dimethoxy- 5,6-dihydrobenzo [g]- 1,3-benzodioxolo [5,6-a] quinolizinium chloride (Compound #1 salt).

**Table 1**

| **Tumour type** | **Cell line /Drug** | **Compound #1 IC₅₀ (µM)** |
|---|---|---|
| Pleural Mesothelioma | MSTO-211H | 1.15 ± 0.11 |
| | MSTO-21 1 H/5-Fluorouracil resistant | 1.25 ± 0.19 |
| | H28 | 1.11± 0.16 |
| | MPM236 | 0.72 ± 0,07 |
| | MPM236-Pemetrexed resistant | 0.83 ± 0.1 |
| | MPM095 | 1.53 ± 0.15 |
| | MPM095- Pemetrexed/cisplatin resistant | 1.55 ± 0.16 |
| Liver | HepG2 | 4.87 ± 0.20 |
| NSCLC | A549 | 2.79 ± 0.82 |
| SCLC | NCI H23 | 0.86 ± 0.1 |
| Breast | MCF7 | 8.3 ± 2.1 |
| Colon | Caco-2 | 9.54 ± 0.47 |
| | HT29 | 1.39 ± 0.24 |
| | HCT116 | 4.12 ± 1.97 |
| Ovarian | SK-OV3 | 1.5 ± 0,8 |
| | Igrov-1 | 1.0 ± 0.1 |
| | Igrov-1/cisplatin Resistant | 0.85 ± 0.4 |
| Leukaemia | HL60 | 0.16 ± 0.1 |

Table 1 above shows cytotoxicity data (IC50) of Compound #1 determined by colorimetric MTT Assay performed in a panel of cancer cell lines, including cell lines resistant to their respective Standard of Care treatment.

The data shown in Figures 1A to 1D and in Table 1 demonstrates significant inhibition of cell viability across a range of cancer types including solid tumours (preferably mesothelioma, non-small cell lung cancer, colon cancer, ovarian cancer), including resistant cancer types (see for example pleural mesothelioma and ovarian), and haematological tumours (leukaemia) at very small doses. This demonstrates the potential of Compound #1 as an anticancer agent, particularly for resistant types of cancer solid tumours.

Without being particularly bound by theory, Compound #1 seems to function as a post-transcriptional regulator of the Thymidylate Synthase (TS) gene by directly binding to complex secondary structures present in the 5' untranslated region (5' UTR) of TS mRNA, for example hairpin loops or G-quadruplexes, effectively silencing the expression of this protein in tumours, based on different in-vitro assay for measuring gene and protein expression that have been performed (data not shown). Under normal conditions, the TS protein in its ligand-free form binds to these elements in its own mRNA, thereby repressing its translation and controlling TS levels within the cell. However, in tumour cells, where rapid proliferation demands higher TS production, all available TS is quickly utilized, leading to overproduction and a loss of the protein's ability to effectively regulate its mRNA. Consequently, these regulatory sites in the 5' UTR become ideal targets for therapeutic intervention and Compound #1 can bind to TS mRNA and replicate the normal regulatory function of the TS protein, thus restoring control over TS translation and providing an alternative mechanism to regulate TS expression in tumour cells. By potentially binding specifically to these RNA complex secondary structures, Compound #1 inhibits the translation of the TS protein, thereby disrupting an essential pathway on which cancer cells rely. This targeted approach contrasts sharply with traditional therapies that broadly affect all rapidly dividing cells, enhancing therapeutic precision and potentially lowering overall toxicity, and may explain the results obtained in resistant cell lines and the ability to synergize with standard of care treatments. For example, compared to current clinically used TS inhibitors such as pemetrexed that bind the protein and inhibits its activity, Compound #1 binds directly to mRNA and silences the expression of TS enzyme in the tumour while overcoming resistance associated with certain drugs commonly used to treat solid tumours. This mechanism may be the same for compounds closely related to Compound 1# based on their structural features, particularly the combination of the berberine ring structure combined with the diphenyl propyl substituent in position C13.

### Example 3

### Orthotopic MSTO-211H pleural mesothelioma model

Compound #1 was tested *in vivo* in pleural mesothelioma orthotopic (surgically implanted tumour in pleural tissue) xenograft models with MSTO-211H. Intravenous (i.v.) administration of Compound #1 alone or in combination with cisplatin was performed.

The treatment with Compound #1, either as a monotherapy or in combination with cisplatin, resulted in a significant *in vivo* reduction in tumor growth compared to the control and standard of care (pemetrexed + cisplatin) groups as shown in Figure 2 panel A. Body weight measurements indicate no significant signs of toxicity, with stable animal weight observed throughout the study period as shown in Figure 2 panel B. The combination therapy demonstrated a synergistic effect, leading to delayed tumor progression without major safety concerns. At tissue level, histological analysis correlated treatment with compound 1# with necrosis associated with tumour reduction (data not shown).

In a further *in vivo* study performed in an orthotopic MSTO-211H pleural mesothelioma model (data not shown), the addition of Compound 1# to the standard of care cisplatin therapy showed a clear benefit in tumour growth delay, while proved synergistic effects allowing to reduce the dose of Compound #1 *in vivo* and thus, reducing potential toxic side-effects due to the drug. The delay in tumour growth was maintained after completing the treatment. No major signs of toxicity or safety concerns were found.

### Example 4

### NSCLC Xenograft Model model

Compound #1 was tested in a in A549 induced-mouse model (xenograft) of non-small cell lung cancer (NSCLC). The antitumor efficacy of Compound #1 was tested by intravenous (i.v.) route with twice weekly administrations for four weeks in A549 induced-mouse model (xenograft) of lung cancer in comparison with i.v. administration of cisplatin.

As shown in Figures 3A Compound #1 shows a dose-dependent efficacy, with the 3 mg/kg dose showing a 47% inhibition of tumor growth, which was statistically significant when compared to active compound cisplatin. No toxic effects were observed in animal body weight remained stable throughout the study, indicating a favorable safety profile (Fig. 3B). The *in vivo* potency of Compound #1 was comparable to, or exceeded, that of cisplatin, confirming its potential as a more effective and safer treatment option for lung cancer.

Intravenous administration presents challenges both from a formulation point of view of the compound as well as from an *in vivo* efficacy and a safety perspective, e.g. the risk of reduced efficacy due problems during preparation of the injectable formulation or potential side effects derived from the compound being delivered directly into the blood stream. To the best knowledge of the inventors, efficacious intravenous administration to treat cancer, in particular solid tumours, has not been proved before for compounds such as Compound 1#. Thus, the examples above show how Compound 1# and likely closely related compounds can exert its antitumoral activity in vivo and upon intravenous administration, particularly against solid tumours, and demonstrates that Compound 1# can safely achieve its therapeutical effect against a variety of cancers via this administration route, thus allowing therapeutic treatments that have the advantages of such administration route such as the compound rapidly reaching its target and the therapeutic effect being achieved quickly.

### Example 5

### AOM/DSS colorectal carcinogenesis model

AOM/DSS model is a well-established system for studying inflammation-driven colorectal carcinogenesis. Compound #1 (3mg/kg) reduced significantly the number of tumours *in vivo* as compared to untreated animals. The number of tumours were counted after experiment termination. Body weight measurements indicate no significant signs of toxicity, with stable animal weight observed throughout the study period.

The project leading to these Examples has received funding from the European Union's Programme for Research and Innovation Horizon 2020 (Grant Agreement ID 829416), funding from the European Union's Horizon 2020 research and innovation programme under the Marie Sktodowska-Curie (Grant Agreement No 766214) and funding from the CDTI as part of the programme to support SMEs with the Seal of Excellence of the Horizon Europe EIC Accelerator (SoE2022-1025).

## Claims

1. A compound of general formula (I) 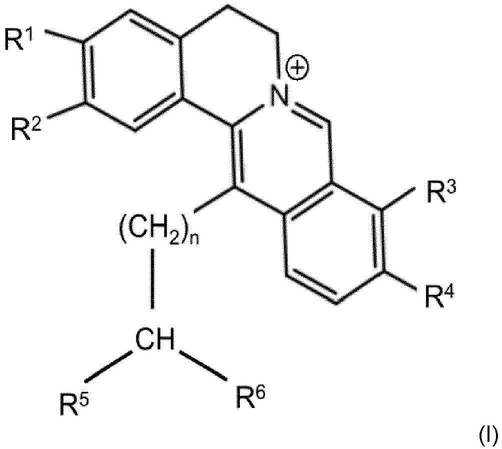 wherein
R1 and R2 taken together are a methylenedioxy group, R3 is a methoxy group, R4 is a methoxy group, R5 is a phenyl group, and R6 is a phenyl group; or wherein
R1 and R2 taken together are a methylenedioxy group, R3 is a methoxy group, R4 is a hydroxy group, R5 is a phenyl group, and R6 is a phenyl group; or wherein
R1 and R2 taken together are a methylenedioxy group, R3 is a hydroxy group, R4 is a methoxy group, R5 is a phenyl group, and R6 is a phenyl group; or wherein
R1 is a methoxy group, R2 is a hydroxy group, R3 is a methoxy group, R4 is a methoxy group, R5 is a phenyl group, and R6 is a phenyl group; or wherein
R1 is a hydroxy group, R2 is a methoxy group, R3 is a methoxy group, R4 is a methoxy group, R5 is a phenyl group, and R6 is a phenyl group, and wherein
n is 2;
for use in the effective treatment of cancer in a human subject, wherein said cancer comprises or consists of an advanced tumour, wherein said advanced tumour is a tumour that is at a stage where it has grown to a size which makes it not susceptible to be treated by surgery, and/or it has spread to nearby tissues, and/or it has metastasized to distant parts of the body, and wherein said compound of general formula (I) is administered in a therapeutically effective dose to said human subject, and wherein said compound of general formula (I) effectively treats said cancer by directly binding to complex secondary structures present in the 5' untranslated region (5' UTR) of the Thymidylate Synthase mRNA, for example hairpin loops or G-quadruplexes, effectively silencing the expression of Thymidylate Synthase in said tumour.

2. The compound for use according to claim 1, wherein said compound is administered intravenously.

3. The compound for use according to any one of the preceding claims, wherein said cancer comprises or consists of a solid tumour.

4. The compound for use according to any one of the preceding claims, wherein said cancer comprises or consists of a tumour selected from the group consisting of a refractory tumour, a resistant tumour, a relapsed/recurrent tumour, and a combination thereof.

5. The compound for use according to any one of the preceding claims, wherein said cancer comprises or consists of an advanced refractory solid tumour or an advanced resistant solid tumour.

6. The compound for use according to any one of claims 3 to 5, wherein said cancer is mesothelioma, preferably pleural mesothelioma or peritoneal mesothelioma, and more preferably pleural mesothelioma.

7. The compound for use according to any one of claims 3 to 5, wherein said cancer is a lung cancer, preferably wherein said lung cancer is a small cell lung cancer or a non-small-cell lung cancer, more preferably wherein said lung cancer is a non-small cell lung cancer subtype, and even more preferably wherein said lung cancer is a non-small cell lung cancer subtype selected from the group consisting of adenocarcinoma and a squamous cell carcinoma.

8. The compound for use according to any one of claims 3 to 5, wherein said cancer is colorectal cancer.

9. The compound for use according to any one of claims 3 to 5, wherein said cancer is ovarian cancer.

10. The compound for use according to any one of the preceding claims, wherein said treatment is administered in combination with an additional cancer therapy, preferably wherein said additional cancer therapy is selected from the group consisting of chemotherapy, radiotherapy, immunotherapy, hormonal therapy, and a combination thereof, and more preferably wherein said additional cancer therapy is the cancer therapy considered the standard of care treatment for said cancer, and even more preferably said additional cancer therapy is cisplatin.

11. A pharmaceutical composition for use according to any one of claims 1 to 10, **characterized in that** said pharmaceutical composition comprises a compound of general formula I as defined in claim 1, and **in that** said pharmaceutical composition is administered to said human subject such that a therapeutically effective dose of said compound of general formula (I) is administered to said human subject.

12. The pharmaceutical composition for use according to claim 11, wherein said pharmaceutical composition is a liquid pharmaceutical composition comprising a pharmaceutically acceptable vehicle such that said liquid pharmaceutical composition is isotonic and such that said liquid pharmaceutical composition has a physiologic pH value, and preferably wherein said pharmaceutically acceptable vehicle is a phosphate buffer.

13. The pharmaceutical composition for use according to claim 11 or 12, wherein said pharmaceutical composition is in the form of a lyophilized powder for reconstitution and for injection in humans, preferably via intravenous route.

## Patentansprüche

1. Verbindung der allgemeinen Formel (I) 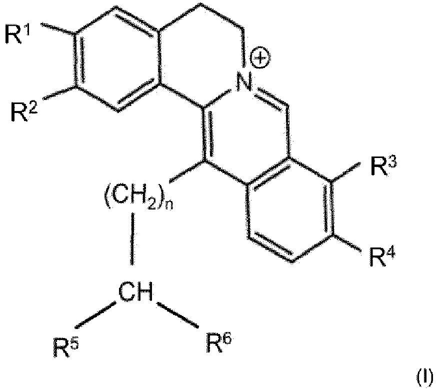 wobei
R1 und R2 zusammen eine Methylendioxygruppe bilden, R3 eine Methoxygruppe ist, R4 eine Methoxygruppe ist, R5 eine Phenylgruppe ist und R6 eine Phenylgruppe ist; oder wobei
R1 und R2 zusammen eine Methylendioxygruppe bilden, R3 eine Methoxygruppe ist, R4 eine Hydroxygruppe ist, R5 eine Phenylgruppe ist und R6 eine Phenylgruppe ist; oder wobei
R1 und R2 zusammen eine Methylendioxygruppe bilden, R3 eine Hydroxygruppe ist, R4 eine Methoxygruppe ist, R5 eine Phenylgruppe ist und R6 eine Phenylgruppe ist; oder wobei
R1 eine Methoxygruppe ist, R2 eine Hydroxygruppe ist, R3 eine Methoxygruppe ist, R4 eine Methoxygruppe ist, R5 eine Phenylgruppe ist und R6 eine Phenylgruppe ist; oder wobei
R1 eine Hydroxygruppe ist, R2 eine Methoxygruppe ist, R3 eine Methoxygruppe ist, R4 eine Methoxygruppe ist, R5 eine Phenylgruppe ist und R6 eine Phenylgruppe ist, und wobei
n gleich 2 ist;
zur Verwendung bei der wirksamen Behandlung von Krebs bei einem Menschen, wobei der Krebs einen fortgeschrittenen Tumor umfasst oder aus einem fortgeschrittenen Tumor besteht, wobei der fortgeschrittene Tumor ein Tumor ist, der sich in einem Stadium befindet, in dem er auf eine Größe gewachsen ist, die eine chirurgische Behandlung unmöglich macht, und/oder sich auf benachbarte Gewebe ausgebreitet hat und/oder in entfernten Körperregionen metastasiert hat und wobei die Verbindung der allgemeinen Formel (I) dem Menschen in einer therapeutisch wirksamen Dosis verabreicht wird und wobei die Verbindung der allgemeinen Formel (I) den Krebs wirksam behandelt, indem sie direkt an komplexe Sekundärstrukturen bindet, die in der 5'-untranslatierten Region (5'-UTR) der Thymidylatsynthase-mRNA vorhanden sind, beispielsweise Haarnadelschleifen oder G-Quadruplexe, wodurch die Expression der Thymidylatsynthase in dem Tumor wirksam unterdrückt wird.

2. Verbindung zur Verwendung gemäß Anspruch 1, wobei die Verbindung intravenös verabreicht wird.

3. Verbindung zur Verwendung gemäß einem der vorhergehenden Ansprüche, wobei der Krebs einen soliden Tumor umfasst oder aus einem soliden Tumor besteht.

4. Verbindung zur Verwendung gemäß einem der vorhergehenden Ansprüche, wobei der Krebs einen Tumor umfasst oder aus einem Tumor besteht, ausgewählt aus der Gruppe bestehend aus einem refraktären Tumor, einem resistenten Tumor, einem rezidivierten/wiederkehrenden Tumor und einer Kombination davon.

5. Verbindung zur Verwendung gemäß einem der vorhergehenden Ansprüche, wobei der Krebs einen fortgeschrittenen refraktären soliden Tumor oder einen fortgeschrittenen resistenten soliden Tumor umfasst oder aus einem solchen besteht.

6. Verbindung zur Verwendung gemäß einem der Ansprüche 3 bis 5, wobei der Krebs ein Mesotheliom ist, vorzugsweise ein Pleuramesotheliom oder ein Peritonealmesotheliom, und besonders bevorzugt ein Pleuramesotheliom.

7. Verbindung zur Verwendung gemäß einem der Ansprüche 3 bis 5, wobei der Krebs ein Lungenkrebs ist, wobei der Lungenkrebs bevorzugt ein kleinzelliges Lungenkarzinom oder ein nicht-kleinzelliges Lungenkarzinom ist, wobei der Lungenkrebs besonders bevorzugt ein Subtyp des nicht-kleinzelligen Lungenkarzinoms ist, und wobei der Lungenkrebs am meisten bevorzugt ein Subtyp des nicht-kleinzelligen Lungenkarzinoms ist, ausgewählt aus der Gruppe bestehend aus Adenokarzinom und einem Plattenepithelkarzinom.

8. Verbindung zur Verwendung gemäß einem der Ansprüche 3 bis 5, wobei der Krebs Darmkrebs ist.

9. Verbindung zur Verwendung gemäß einem der Ansprüche 3 bis 5, wobei der Krebs Eierstockkrebs ist.

10. Die Verbindung zur Verwendung gemäß einem der vorhergehenden Ansprüche, wobei die Behandlung in Kombination mit einer zusätzlichen Krebstherapie verabreicht wird, wobei bevorzugt die zusätzliche Krebstherapie aus der Gruppe ausgewählt ist, bestehend aus Chemotherapie, Strahlentherapie, Immuntherapie, Hormontherapie und einer Kombination davon, und besonders bevorzugt, wobei die zusätzliche Krebstherapie die Krebstherapie ist, die als Standardtherapie für den Krebs gilt, und am meisten bevorzugt, wobei die zusätzliche Krebstherapie Cisplatin ist.

11. Pharmazeutische Zusammensetzung zur Verwendung gemäß einem der Ansprüche 1 bis 10, **dadurch gekennzeichnet, dass** die pharmazeutische Zusammensetzung eine Verbindung der allgemeinen Formel I, wie in Anspruch 1 definiert, umfasst, und dass die pharmazeutische Zusammensetzung dem Menschen so verabreicht wird, dass dem Menschen eine therapeutisch wirksame Dosis der Verbindung der allgemeinen Formel (I) verabreicht wird.

12. Pharmazeutische Zusammensetzung zur Verwendung gemäß Anspruch 11, wobei die pharmazeutische Zusammensetzung eine flüssige pharmazeutische Zusammensetzung ist, die einen pharmazeutisch akzeptablen Träger umfasst, so dass die flüssige pharmazeutische Zusammensetzung isotonisch ist und dass die flüssige pharmazeutische Zusammensetzung einen physiologischen pH-Wert aufweist, und wobei der pharmazeutisch akzeptable Träger vorzugsweise ein Phosphatpuffer ist.

13. Pharmazeutische Zusammensetzung zur Verwendung gemäß Anspruch 11 oder 12, wobei die pharmazeutische Zusammensetzung in Form eines gefriergetrockneten Pulvers zur Rekonstitution und zur Injektion bei Menschen, vorzugsweise intravenös, vorliegt.

## Revendications

1. Composé de formule générale (I) 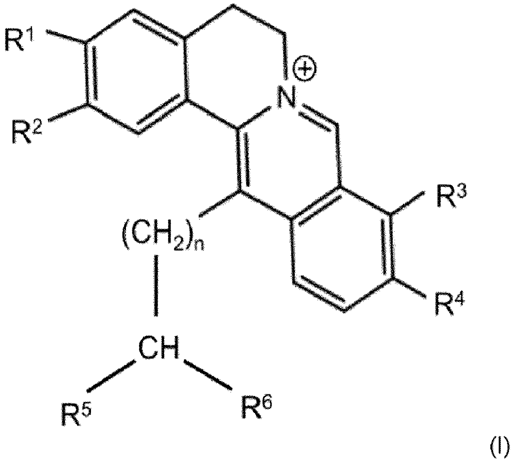 dans lequel
R1 et R2 pris ensemble sont un groupe méthylènedioxy, R3 est un groupe méthoxy, R4 est un groupe méthoxy, R5 est un groupe phényle, et R6 est un groupe phényle ; ou dans lequel
R1 et R2 pris ensemble sont un groupe méthylènedioxy, R3 est un groupe méthoxy, R4 est un groupe hydroxy, R5 est un groupe phényle, et R6 est un groupe phényle ; ou dans lequel
R1 et R2 pris ensemble sont un groupe méthylènedioxy, R3 est un groupe hydroxy, R4 est un groupe méthoxy, R5 est un groupe phényle, et R6 est un groupe phényle ; ou dans lequel
R1 est un groupe méthoxy, R2 est un groupe hydroxy, R3 est un groupe méthoxy, R4 est un groupe méthoxy, R5 est un groupe phényle, et R6 est un groupe phényle ; ou dans lequel
R1 est un groupe hydroxy, R2 est un groupe méthoxy, R3 est un groupe méthoxy, R4 est un groupe méthoxy, R5 est un groupe phényle, et R6 est un groupe phényle, et dans lequel
n vaut 2 ;
pour une utilisation dans le traitement efficace d'un cancer chez un sujet humain, dans lequel ledit cancer comprend ou consiste en une tumeur avancée, dans lequel ladite tumeur avancée est une tumeur qui est à un stade où elle a atteint une taille qui la rend insensible au traitement par chirurgie, et/ou elle s'est propagée aux tissus voisins, et/ou elle s'est métastasée à des parties éloignées du corps, et dans lequel ledit composé de formule générale (I) est administré à une dose thérapeutiquement efficace audit sujet humain, et dans lequel ledit composé de formule générale (I) traite efficacement ledit cancer en se liant directement à des structures secondaires complexes présentes dans la région non traduite 5' (5' UTR) de l'ARNm de thymidylate synthase, par exemple des boucles d'épingles à cheveux ou des G-quadruplexes, silenciant efficacement l'expression de thymidylate synthase dans ladite tumeur.

2. Composé pour son utilisation selon la revendication 1, dans lequel ledit composé est administré par voie intraveineuse.

3. Composé pour son utilisation selon l'une quelconque des revendications précédentes, dans lequel ledit cancer comprend ou consiste en une tumeur solide.

4. Composé pour son utilisation selon l'une quelconque des revendications précédentes, dans lequel ledit cancer comprend ou consiste en une tumeur choisie dans le groupe consistant en une tumeur réfractaire, une tumeur résistante, une tumeur récidivante/récurrente, et une combinaison de celles-ci.

5. Composé pour son utilisation selon l'une quelconque des revendications précédentes, dans lequel ledit cancer comprend ou consiste en une tumeur solide réfractaire avancée ou une tumeur solide résistante avancée.

6. Composé pour son utilisation selon l'une quelconque des revendications 3 à 5, dans lequel ledit cancer est un mésothéliome, de préférence un mésothéliome pleural ou un mésothéliome péritonéal, et plus préférentiellement un mésothéliome pleural.

7. Composé pour son utilisation selon l'une quelconque des revendications 3 à 5, dans lequel ledit cancer est un cancer du poumon, de préférence dans lequel ledit cancer du poumon est un cancer du poumon à petites cellules ou un cancer du poumon non à petites cellules, plus préférentiellement dans lequel ledit cancer du poumon est un sous-type de cancer du poumon non à petites cellules, et encore plus préférentiellement dans lequel ledit cancer du poumon est un sous-type de cancer du poumon non à petites cellules choisi dans le groupe consistant en un adénocarcinome et un carcinome épidermoïde.

8. Composé pour son utilisation selon l'une quelconque des revendications 3 à 5, dans lequel ledit cancer est un cancer colorectal.

9. Composé pour son utilisation selon l'une quelconque des revendications 3 à 5, dans lequel ledit cancer est un cancer de l'ovaire.

10. Composé pour son utilisation selon l'une quelconque des revendications précédentes, dans lequel ledit traitement est administré en combinaison avec une thérapie anticancéreuse supplémentaire, de préférence dans lequel ladite thérapie anticancéreuse supplémentaire est choisie dans le groupe consistant en chimiothérapie, radiothérapie, immunothérapie, hormonothérapie et une combinaison de celles-ci, et plus préférentiellement dans lequel ladite thérapie anticancéreuse supplémentaire est la thérapie anticancéreuse considérée comme le traitement de référence pour ledit cancer, et encore plus préférentiellement, ladite thérapie anticancéreuse supplémentaire est le cisplatine.

11. Composition pharmaceutique pour son utilisation selon l'une quelconque des revendications 1 à 10, **caractérisée en ce que** ladite composition pharmaceutique comprend un composé de formule générale I tel que défini dans la revendication 1, et **en ce que** ladite composition pharmaceutique est administrée audit sujet humain de sorte qu'une dose thérapeutiquement efficace dudit composé de formule générale (I) soit administrée audit sujet humain.

12. Composition pharmaceutique pour son utilisation selon la revendication 11, dans laquelle ladite composition pharmaceutique est une composition pharmaceutique liquide comprenant un véhicule pharmaceutiquement acceptable de sorte que ladite composition pharmaceutique liquide soit isotonique et de sorte que ladite composition pharmaceutique liquide présente une valeur de pH physiologique, et de préférence dans laquelle ledit véhicule pharmaceutiquement acceptable est un tampon phosphate.

13. Composition pharmaceutique pour son utilisation selon la revendication 11 ou 12, dans laquelle ladite composition pharmaceutique se présente sous la forme d'une poudre lyophilisée pour reconstitution et pour injection chez l'être humain, de préférence par voie intraveineuse.
